# EUROPÄISCHE PATENTSCHRIFT

(11) **EP 0 774 921 B1**
(45) Veröffentlichungstag und Bekanntmachung des Hinweises auf die Patenterteilung: **25.03.1998**
(21) Anmeldenummer: 95926368.2
(22) Anmeldetag: 28.07.1995
(51) Int. Cl.: A61B 6/14

(54) **STRAHLENDIAGNOSEEINRICHTUNG ZUR ERSTELLUNG VON PANORAMASCHICHTAUFNAHMEN**
RADIATION DIAGNOSTIC DEVICE FOR PRODUCING PANORAMIC SECTIONAL IMAGES
DISPOSITIF DE RADIODIAGNOSTIC SERVANT A REALISER DES TOMOGRAPHIES PANORAMIQUES

(30) Priorität: 10.08.1994 DE 4428331
(43) Veröffentlichungstag der Anmeldung: 28.05.1997
(73) Patentinhaber: SIEMENS AKTIENGESELLSCHAFT, 80333 München (DE)
(72) Erfinder: SCHULZE-GANZLIN, Ulrich, D-64653 Lorsch (DE); PLÖTZ, Josef, D-64625 Bensheim (DE)
(86) Internationale Anmeldenummer: DE9500989
(87) Internationale Veröffentlichungsnummer: WO9604849

(56) Entgegenhaltungen:
- EP-A- 0 262 500
- DE-A- 4 133 066
- US-A- 5 228 068

## Beschreibung

Die Erfindung betrifft eine Strahlendiagnoseeinrichtung zur Erstellung von Panoramaschichtaufnahmen mit einer Einrichtung zum tomographischen Abtasten eines Objektes mittels Strahlung und zum Erzeugen von elektrischen Signalen in Abhängigkeit vom Strahlenschatten des Objektes und mit einer Signalverarbeitungseinrichtung aufweisend einen Speicher zum Speichern der elektrischen Signale während der tomographischen Abtastung und aufweisend einen Rechner zum Berechnen von Signalen entsprechend eines Schichtbildes des Objektes in einer gewünschten Schichtlage.

Eine solche, aus der DE 41 33 066 A1 bekannte Strahlendiagnoseeinrichtung weist eine Schwenkeinheit auf, an der, einander gegenüberliegend, eine Röntgenstrahlenquelle und ein Röntgenbilddetektor angeordnet sind. Der Röntgenbilddetektor erfaßt die Röntgenstrahlen, die ein zwischen der Röntgenstrahlenquelle und dem Röntgenbilddetektor angeordnetes Aufnahmeobjekt durchdrungen haben. Die von dem Röntgenbilddetektor erhaltenen Bildinformationen werden, während die Röntgenstrahlenquelle und der Röntgenbilddetektor um das Aufnahmeobjekt gedreht werden, einem Bildspeicher zugeführt, der die erhaltenen Bildinformationen als Einzelbilder sequentiell speichert. Zur Erstellung eines Panoramabildes ist eine Bildverarbeitungseinrichtung vorgesehen, die in vorbestimmbaren Ableitungsintervallen Einzelbilder aus dem Bildspeicher ausliest, diese um einen wählbaren Verschiebebetrag verschiebt und die abgeleiteten und verschobenen Einzelbilder addiert. Die Lage der tomographischen Bildschicht eines so erstellten Panoramabildes ist abhängig vom Intervall für das Ableiten der Bildinformationen aus der Bildspeicheranordnung und abhängig vom Betrag der Verschiebung der Bildinformation zum Zeitpunkt der Addition. Durch Wahl eines anderen Ableitungsintervalles und eines anderen Verschiebebetrages kann ein weiteres Panoramabild in einer weiteren tomographischen Bildschicht berechnet werden. Um unerwünschte, dem ersten Panoramabild überlagerte Auswirkungen zu eliminieren, die beispielsweise auf ein Objekt zurückgehen, das in einer anderen tomographischen Bildschicht liegt, die von der gewünschten tomographischen Bildschicht verschieden ist, ist es bekannt, in einer zweiten Bildschicht ein zweites Panoramabild zu berechnen. Das zweite Panoramabild wird in ein projiziertes Panoramabild umgewandelt, das erzeugt wird, wenn das zweite Panoramabild auf die vorbestimmte tomographische Bildschicht projiziert wird. Dieses projizierte Panoramabild wird von dem ersten Panoramabild subtrahiert, um auf diese Weise ein Panoramabild zu erhalten, das frei von diesen unerwünschten Auswirkungen ist. Es kann somit ein klares und deutliches Panoramabild in der bestimmten tomographischen Bildschicht erstellt werden.

Da nicht davon ausgegangen wird, daß die gewünschte Bildschicht durch Eingabe des hierzu relevanten Ableitungsintervalles und des Verschiebebetrages direkt gefunden wird, ist ein manuelles Näherungsverfahren durch Auswahl unterschiedlicher Ableitungsintervalle und Verschiebebeträge so lange durchzuführen, bis ein Panoramabild in einer gewünschten Bildschicht berechnet ist. Dieses vom Personal durchzuführende Näherungsverfahren ist sehr zeitaufwendig. Zudem ist der Zeitbedarf für die Berechnung der Panoramabilder bei diesen Näherungsverfahren groß. Das Personal muß durch Betrachten der berechneten Panoramabilder die gewünschte Bildschicht selbst bestimmen.

Aufgabe der Erfindung ist es daher, eine Strahlendiagnoseeinrichtung der eingangs genannten Art so auszubilden, daß der Zeitbedarf für die Bestimmung der gewünschten Schichtlage für ein zu berechnendes Panoramabild gering ist.

Die Aufgabe wird erfindungsgemäß bei einer Strahlendiagnoseeinrichtung der eingangs genannten Art dadurch gelöst, daß der Rechner die gewünschte Schichtlage aus der Berechnung von Einzelschichtlagen und deren Auswertung bestimmt.

Vorteil der Erfindung ist, daß somit nicht mehr durch Personal die Ableitungsintervalle und die Verschiebebeträge eingegeben werden müssen, sondern daß der Rechner aufgrund der Auswertung von Einzelbildern die gewünschte, diagnostisch ideale Schichtlage eines Schichtbildes bestimmt. Der Zeitbedarf für die Erstellung eines Panoramabildes in der gewünschten Schichtlage ist damit im wesentlichen nur noch von der Rechenkapazität des Rechners abhängig und ist somit gegenüber den hierzu zeitaufwendigen Handlungen durch Personal erheblich reduziert.

Es ist vorteilhaft, wenn der Rechner die gewünschte Schichtlage als mittlere Schichtlage zwischen einer ersten und einer zweiten Einzelschichtlage berechnet, insbesondere dann, wenn bei einer zahnärztlichen Diagnoseeinrichtung eine Einzelschichtlage durch die Innen- und die zweite Einzelschichtlage durch die Außenseite des Kiefers bzw. eines Zahnes definiert ist. Ausgehend von der somit vom Rechner bestimmten gewünschten Schichtlage können weitere Schichtlagen und damit Panoramabilder des Zahnes oder des Kiefers ohne großen Zeitaufwand berechnet werden.

Besonders vorteilhaft ist es, wenn der Rechner die mittlere Schichtlage durch die Berechnung von Einzelschichtlagen an unterschiedlichen Stellen des Objektes bestimmt. Durch Verbinden der einzelnen mittleren Schichtlagen kann somit eine gemeinsame Schichtlage definiert werden, die als Ausgangspunkt für die Berechnung von gemeinsamen Panoramaschichten dient. Dies gilt insbesondere dann, wenn die gemeinsame mittlere Schichtlage dem Kieferbogen angepaßt ist, so daß Panoramabilder des Kiefers erstellt werden können.

Um den Zeitbedarf für die Erstellung von Panoramakieferaufnahmen weiter zu reduzieren ist es vorteilhaft, wenn in einem weiteren Speicher Signale wenigstens eines durchschnittlichen Kieferbogens gespeichert sind, wenn der Rechner die mittlere Schichtlage an wenigstens drei unterschiedlichen Stellen des Kiefers bestimmt und wenn durch einen Vergleich der mittleren Schichtlage mit dem wenigstens einen gespeicherten Kieferbogen die gewünschte Schichtlage definiert wird. Es können somit auch ohne großen Zeitaufwand Panoramaaufnahmen eines stark von der Norm abweichenden Kiefers erstellt und somit auch die Abweichung von der Norm bestimmt werden.

Weitere Vorteile und Einzelheiten der Erfindung ergeben sich aus der Beschreibung eines Ausführungsbeispieles einer Strahlendiagnoseeinrichtung der eingangs genannten Art anhand der Figuren. Es zeigen:
- FIG 1: eine Strahlendiagnoseeinrichtung nach der Erfindung in prinzipieller Darstellung,
- FIG 2: einen Kieferbogen zur Erläuterung der Diagnoseeinrichtung nach FIG 1,
- FIG 3: einen Bereich des Kieferbogens nach FIG 2,

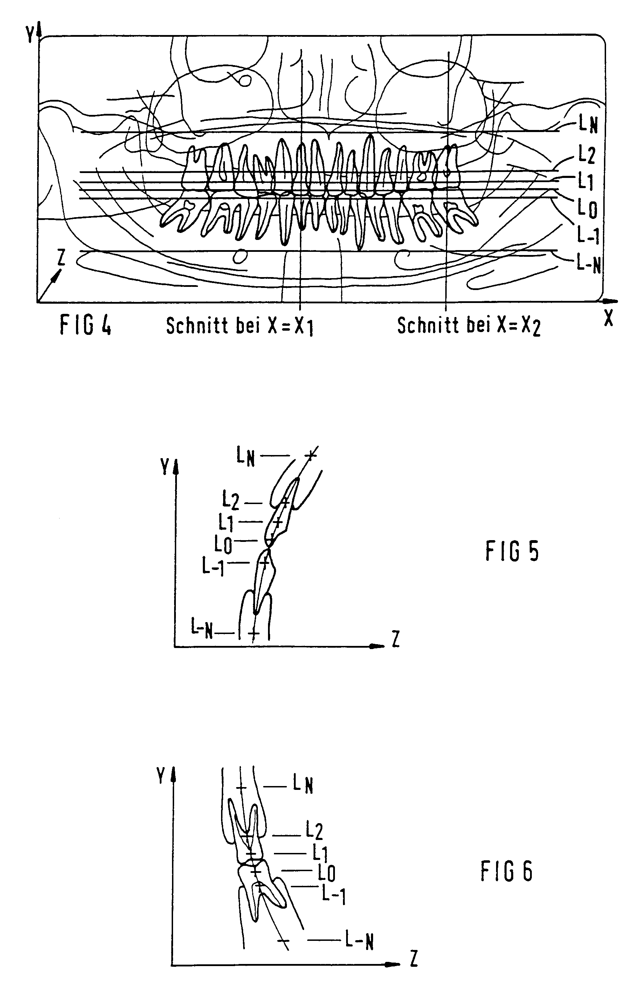

- FIG 4: eine Kieferdarstellung in einer frontalen Ansicht mit Schichtlinien LO-LN,
- FIG 5: eine Seitenansicht eines vorderen Zahnbereiches mit Schichtlinien LO-LN und
- FIG 6: eine Seitenansicht eines Backenzahnbereiches mit Schichtlinien LO-LN.

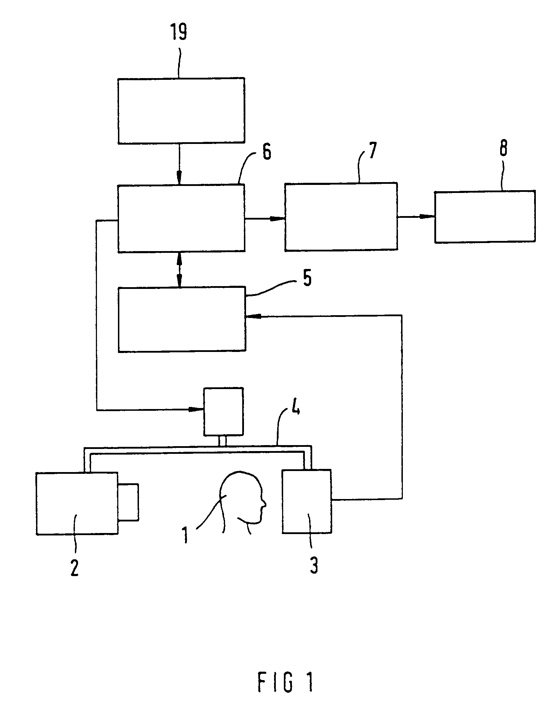

In der FIG 1 ist eine Strahlendiagnoseeinrichtung zur Erstellung von Panoramaschichtaufnahmen in prinzipieller Weise dargestellt. Hierbei ist eine Einrichtung zum tomographischen Abtasten eines Objektes 1 vorgesehen, die einen Strahlensender 2 und einen Strahlenempfänger 3 aufweist, die einander gegenüberliegend an einer Schwenkeinheit 4 angeordnet sind. Der Strahlensender 2, der beispielsweise als Röntgenstrahlensender ausgeführt ist, kann durch entsprechende Ansteuerung und Einblendmittel vorzugsweise ein schlitzförmiges Strahlenbündel senden, das das Objekt 1 durchdringt und als Strahlenschatten des Objektes 1 auf den Strahlenempfänger 3 auftrifft, der, dem Strahlenschatten entsprechend, elektrische Signale erzeugt. Der Strahlenempfänger 3 kann hierzu als Bildverstärker ausgeführt sein, dem eine Fernsehkamera oder ein elektrooptischer Lichtwandler, z.B. ein CCD, nachgeschaltet ist. Ebenso ist es möglich, einen Strahlenwandler vorzusehen, der aufgrund von auftreffender Strahlung entsprechende elektrische Signale direkt erzeugt oder Licht erzeugt, das von einer Fernsehkamera oder einem CCD-Wandler erfaßt und in entsprechende elektrische Signale gewandelt wird. Während der tomographischen Abtastung werden die elektrischen Signale in einen Speicher 5 eingelesen, der somit Signale entsprechend einer Folge von Einzelbildern enthält. Ein Rechner 6 ist vorgesehen zum Steuern der Schwenkeinheit 4 und zum Berechnen von Signalen entsprechend eines Schichtbildes des Objektes 1 in einer gewünschten Schichtlage aufgrund der im Speicher 5 gespeicherten Signale. Dem Rechner 6 kann ein zweiter Speicher 7 zum Speichern von Signalen entsprechend wenigstens einem Schichtbild des Objektes 1 in einer gewünschten Schichtlage nachgeschaltet sein. Eine Ausgabeeinheit 8 zum Erzeugen eines sichtbaren Schichtbildes des Objektes 1 ist entweder direkt dem Rechner 6 oder dem zweiten Speicher 7 nachgeschaltet.

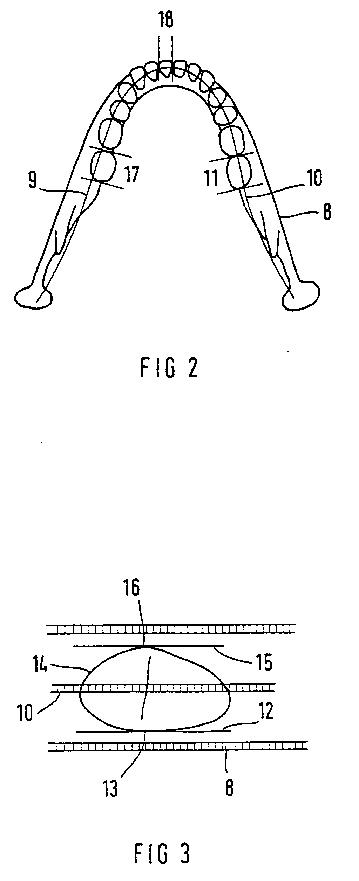

Gemäß der Erfindung ist vorgesehen, daß der Rechner 6 die gewünschte Schichtlage aus der Berechnung von Einzelschichtlagen und deren Auswertung bestimmt. Zur Erläuterung der Erfindung wird nachfolgend auf die FIG 2 und 3 verwiesen. In der FIG 2 ist ein Kiefer 8 eines Objektes 1 in einer Draufsicht gezeigt. Der Kieferbogen ist mit dem Bezugszeichen 9 gekennzeichnet. Soll dieser Kieferbogen 9 als gewünschte Schichtlage 10 eines individuellen Aufnahmevorgangs definiert sein, in der ein Schichtbild erstellt werden soll, so gilt es, diese ohne großen Zeitaufwand aus den Signalen im Speicher 5 zu berechnen. Gemäß der Erfindung ist daher vorgesehen, daß der Rechner 6 die einem vorbestimmten Bereich 11 entsprechenden Signale aus dem Speicher 5 ausliest und Einzelschichtlagen berechnet und auswertet. Eine erste Einzelschichtlage 12 kann beispielsweise dadurch definiert sein, daß sie die Innenseite 13 eines Zahnes 14 tangiert. Die Definition der ersten Einzelschichtlage 12 kann beispielsweise dadurch erfolgen, daß der Rechner 6 ein erstes und zweites Schichtbild des Bereiches 11 in einer ersten und zweiten Schichtlage berechnet und aufgrund eines Vergleiches dieser Schichtbilder hinsichtlich einer charakteristischen Strahlenabsorption des Objektes 1 feststellt, ob sich die zweite Schichtlage näher an der Innenseite 13 des Zahnes befindet als die erste Schichtlage. Ist dies der Fall, so berechnet der Rechner 6 ein drittes Schichtbild des Bereiches 11 in einer dritten Schichtlage und vergleicht, ob sich die dritte Schichtlage näher an der Innenseite 13 des Zahnes 14 befindet als die zweite Schichtlage. Diese Berechnung wird solange fortgesetzt, bis die erste Einzelschichtlage 12 gefunden ist. Analog hierzu erfolgt die Bestimmung der zweiten Einzelschichtlage 15, die die Außenseite 16 des Zahnes 14 tangiert. Analog hierzu können aber auch eine erste und zweite Einzelschichtlage berechnet werden, die den Kiefer 8 tangieren. Das vorbeschriebene Näherungsverfahren zum Definieren der Einzelschichtlagen 12,15 kann nicht nur mit dem Rechner 6 sondern auch in Verbindung mit oder durch eine Fuzzy-Logik durchgeführt werden. Die gewünschte Schichtlage 10 kann als Referenzschichtlage für weitere Schichtlagen beispielsweise als mittlere Schichtlage zwischen der ersten und zweiten Einzelschichtlage 12,15 definiert sein. Die Berechnung der mittleren Schichtlage 10 für den Bereich 11 ist wenig zeitintensiv, da hierzu nur die elektrischen Signale herangezogen werden, die dem Bereich 11 zugeordnet sind. Analog hierzu kann die mittlere Schichtlage 10 in den weiteren Bereichen 17 und 18 berechnet werden.

Ist dem Rechner 6 ein dritter Speicher 19 zugeordnet, in dem Signale entsprechend einer Schichtlage eines durchschnittlichen Kieferbogens gespeichert sind, so kann aufgrund der mittleren Schichtlage 10 in wenigstens zwei vorzugsweise drei Bereichen 11,17 und 18 der durchschnittliche Kieferbogen an den Kieferbogen des Objektes 1 über die gesamte Länge des Kiefers 8 angepaßt und somit die gewünschte Schichtlage 10 definiert werden. Im Rahmen der Erfindung können im Speicher 19 auch Signale entsprechend mehrerer Schichtlagen und auch in Abhängigkeit von Alter, Geschlecht, Größe und Gesichtstyp gespeichert sein.

Alternativ kann die gewünschte Schichtlage 10 aber auch dadurch bestimmt werden, daß der Rechner 6 aufgrund von Einzelschichtbildberechnung und Vergleich die Schichtlage der Wurzelspitze und/oder des Nervenkanales eines oder mehrerer Zähne berechnet und somit die gewünschte Schichtlage 10 definiert.

Im Rahmen der Erfindung kann die mittlere Schichtlage 10 nicht nur in drei sondern in beliebig vielen Bereichen des Kiefers 8 ermittelt werden. Durch Verbinden dieser einzelnen Schichtlagen kann die gewünschte Schichtlage 10 definiert werden.

Die vorherige Erläuterung bezieht sich, aus Gründen einer vereinfachten Erläuterung, auf die Berechnung einer gewünschten Schichtlage 10, die sich entlang des Kiefers 8, also zumindest in einer annähernd horizontalen Richtung erstreckt. Tatsächlich wird hierbei allerdings nur eine einzelne Schichtlinie als eindimensionale Größe berechnet. Idealer Weise verläuft die mittlere Schichtlinie durch die okklusale Ebene des Kiefers 8, die die Kaufläche beschreibt. Ausgehend von der mittleren Schichtlinie können weitere Schichtlinien berechnet werden, die in einem dreidimensionalen Raum entlang der Y- Richtung zueinander versetzt sind wenn die okklusale Ebene durch die X- und Z-Richtung definiert ist. Aus der Summe aller Schichtlinien ergibt sich dann eine zweidimensionale Schichtfläche. Bei den bisherigen Betrachtungen wird davon ausgegangen, daß alle berechneten Schichtlinien die gleiche Form haben und die somit gebildete Schichtfläche in Y-Richtung eben ist, damit also im wesentlichen zylinderförmig.

Bei der Abtastung eines Objektes mittels eines Strahlenbündels in Panormaschichtgeräten ist die tatsächliche Schichtfläche aber nicht immer zylinderförmig, sondern vielfach eher in Form eines Segmentes eines Kegelstumpfs mit einem Öffnungswinkel von ca. 7° ausgebildet. Zur Anpassung eines solchen kegelstumpfförmigen Segmentes an die tatsächliche Schichtfläche des Kiefers 8 hat es sich als vorteilhaft erwiesen, wenn der Kopf bei der Erstellung einer Röntgenaufnahme leicht nach vorne geneigt ist.

Der Kiefer 8 ist aber selbst auch nicht in einer Schichtfläche angeordnet, die sich exakt auf eine Ebene abrollen ließe. Es ist in einer weiteren Verallgemeinerung daher von einer auch in Y-Richtung gewölbten Schichtfläche auszugehen. Zum Erhalt einer solchen gewölbten Schichtfläche werden mehrere, in Y-Richtung zueinander versetzte und unterschiedliche Verläufe einnehmende Schichtlinien berechnet und zusammengefaßt, so daß hieraus eine Schichtfläche erhalten wird, die den anatomischen Gegebenheiten sowie etwaigen Unvollkommenheiten bei der Objektpositionierung gerecht wird und die z.B. der Form eines Kugelsegmentes ähneln kann.

Die Schichtlinien LO-LN können, wie bereits zur Berechnung der Schichtlagen ausgeführt, aufgrund von Daten berechnet werden, die bei der Abtastung des Objektes gewonnen und die vom Rechner hinsichtlich der Strahlenabsorption (hohe, geringe oder vorbestimmte Absorption) ausgewertet werden. Eine Mustererkennung, beispielsweise die der Absorption von Zähnen, der Kiefersubstanz, Weichteile und Luft kann ebenfalls hierzu herangezogen werden. Aufgrund dieser Daten wird also ein individueller Kieferatlas in Form eines Datensatzes erhalten, aufgrund dessen die räumliche Lage von Unter- und/oder Oberkiefer und/oder aller Zähne definiert ist.

Im Rahmen der Erfindung kann ausgehend von einer mittleren Schichtlinie LO in Verbindung mit Schichtlinien entsprechend vorgegebener Geometrien (Kugel, Kegel, Modellkiefer) die als Daten in einem Speicher gespeichert sind, eine Berechnung hinsichtlich einer Schichtfläche erfolgen, die dem Aufnahmeobjekt möglichst nahe kommt.

Eine detaillierte Berechnung der Schichtfläche des Aufnahmeobjektes kann aber auch dadurch erfolgen, daß aufgrund des Datensatzes jede einzelne Schichtlinie LO-LN berechnet wird die dann die tatsächliche Schichtfläche definieren.

Aufgrund der Berechnung der Schichtlinien LO-LN werden Daten entsprechend jeweils zugeordneter Koordinaten erhalten aufgrund deren in Verbindung mit dem Ableitungsintervall und dem Verschiebebetrag Signale berechnet werden, aufgrund deren die gewünschte Panoramaschicht auf einem Monitor darstellbar. ist.

## Patentansprüche

1. Strahlendiagnoseeinrichtung zur Erstellung von Panorama-Schichtaufnahmen
mit einer Einrichtung (2,3,4) zum tomographischen Abtasten eines Objektes (1) mittels Strahlung und zum Erzeugen von elektrischen Signalen in Abhängigkeit vom Strahlenschatten des Objektes (1) und
mit einer Signalverarbeitungseinrichtung aufweisend einen Speicher (5) zum Speichern der elektrischen Signale während der tomographischen Abtastung und aufweisend einen Rechner (6) zum Berechnen von Signalen entsprechend eines Schichtbildes des Objektes (1) in einer gewünschten Schichtlage (10),
**dadurch gekennzeichnet**,
daß der Rechner (6) die gewünschte Schichtlage (10) aus der Berechnung von Einzelschichtlagen(12,15) und deren Auswertung bestimmt .

2. Strahlendiagnoseeinrichtung nach Anspruch 1 ,
**dadurch gekennzeichnet**,
daß der Rechner (6) die gewünschte Schichtlage (10) als mittlere Schichtlage zwischen einer ersten und einer zweiten Einzelschichtlage (12,15) berechnet.

3. Strahlendiagnoseeinrichtung nach Anspruch 1 oder 2 , die als zahnärztliche Diagnoseeinrichtung zur Erstellung von Panorama-Schichtaufnahmen eines Kiefers (8) ausgebildet ist, wobei die erste Einzelschichtlage durch die Innen- und die zweite Einzelschichtlage (16) durch die Außenseite des Kiefers (8) definiert ist.

4. Strahlendiagnoseeinrichtung nach Anspruch 1 oder 2 , die als zahnärztliche Diagnoseeinrichtung zur Erstellung von Panorama-Schichtaufnahmen eines Kiefers (8) ausgebildet ist, wobei die erste Einzelschichtlage (12) durch die Innen- (13) und die zweite Einzelschichtlage (15) durch die Außenseite (16) eines Zahnes (14) definiert ist.

5. Strahlendiagnoseeinrichtung nach einem der Ansprüche 1 bis 4,
wobei der Rechner (6) die mittlere Schichtlage durch die Berechnung von Einzelschichtlagen in einem Bereich (11,17,18) des Objektes (1) bestimmt.

6. Strahlendiagnoseeinrichtung nach einem der Ansprüche 3 bis 5,
wobei die mittlere Schichtlage (10) dem Kieferbogen (9) angepaßt ist.

7. Strahlendiagnoseeinrichtung nach einem der Ansprüche 1 bis 6,
wobei in einem weiteren Speicher (19) Signale wenigstens eines durchschnittlichen Kieferbogens gespeichert sind ,
wobei der Rechner (6) die mittlere Schichtlage in wenigstens zwei unterschiedlichen Bereichen (11,17,18) des Kiefers (8) bestimmt und
wobei durch einen Vergleich der mittleren Schichtlage mit dem gespeicherten Kieferbogen die gewünschte Schichtlage (10) definiert wird.

8. Verfahren zum Betrieb einer Strahlendiagnoseeinrichtung nach einem der Ansprüche 1 bis 7, mit folgenden Verfahrensschritten:
a) tomographisches Abtasten eines Objektes (1) mittels Strahlung und Erzeugen von elektrischen Signalen in Abhängigkeit vom Strahlenschatten des Objektes (1),
b) Speicher der elektrischen Signale während der tomographischen Abtastung,
c) Durchführen eines Näherungsverfahrens durch Berechnen von Einzelschichtlagen und deren Vergleich zur Bestimmung einer gewünschten Schichtlage (10) und
d) Erzeugen eines Schichtbildes des Objektes (1) in der gewünschten Schichtlage.

## Claims

1. Radiation diagnostics installation for producing panoramic tomograms, having a device (2, 3, 4) for tomographically scanning a subject (1) by means of radiation and for generating electrical signals as a function of the radiation shadow of the subject (1), and having a signal-processing device comprising a memory (5) for storing the electrical signals during the tomographic scanning and comprising a computer (6) for calculating signals corresponding to a tomogram of the subject (1) in a desired slice position (10),
characterised in that the computer (6) determines the desired slice position (10) from the calculation of individual slice positions (12, 15) and the evaluation thereof.

2. Radiation diagnostics installation according to claim 1, characterised in that the computer (6) calculates the desired slice position (10) as a central slice position between a first and a second individual slice position (12, 15).

3. Radiation diagnostics installation according to claim 1 or 2 that is designed as a dental diagnostics installation for producing panoramic tomograms of a jaw (8), wherein the first individual slice position is defined by the inside of the jaw (8) and the second individual slice position (16) is defined by the outside of the jaw (8).

4. Radiation diagnostics installation according to claim 1 or 2 that is designed as a dental diagnostics installation for producing panoramic tomograms of a jaw (8), wherein the first individual slice position (12) is defined by the inside (13) of a tooth (14) and the second individual slice position (15) is defined by the outside (16) of a tooth (14).

5. Radiation diagnostics installation according to one of the claims 1 to 4, wherein the computer (6) determines the central slice position by calculating individual slice positions in a region (11, 17, 18) of the subject (1).

6. Radiation diagnostics installation according to one of the claims 3 to 5, wherein the central slice position (10) is matched to the mandibular arch (9).

7. Radiation diagnostics installation according to one of the claims 1 to 6, wherein signals of at least one average mandibular arch are stored in a further memory (19), wherein the computer (6) determines the central slice position in at least two different regions (11, 17, 18) of the jaw (8), and wherein the desired slice position (10) is defined by comparing the central slice position with the stored mandibular arch.

8. Method for the operation of a radiation diagnostics installation according to one of the claims 1 to 7, having the following method steps:
a) tomographic scanning of a subject (1) by means of radiation and generation of electrical signals as a function of the radiation shadow of the subject (1);
b) storage of the electrical signals during the tomographic scanning;
c) implementation of an approximation method by calculating individual slice positions and comparing them for the purpose of determining a desired slice position (10); and
d) generation of a tomogram of the subject (1) in the desired slice position.

## Revendications

1. Dispositif de diagnostic radiologique destiné à réaliser des tomographies panoramiques, comportant
un dispositif (2, 3, 4) destiné à l'exploration tomographique d'un objet (1) au moyen d'un rayonnement et à la génération de signaux électriques en fonction de l'ombre de rayonnement de l'objet (1), et
un dispositif de traitement de signaux comportant une mémoire (5) pour mémoriser les signaux électriques pendant l'exploration tomographique et un ordinateur (6) pour calculer des signaux correspondant à une tomographie de l'objet (1) dans une position de couche souhaitée (10),
caractérisé par le fait que
l'ordinateur (6) définit la position de couche (10) souhaitée à partir du calcul de positions de couches individuelles (12, 15) et de leur exploitation.

2. Dispositif de diagnostic radiologique suivant la revendication 1,
caractérisé par le fait que
l'ordinateur (6) calcule la position de couche (10) souhaitée en tant que position de couche moyenne entre une première et une deuxième positions de couche (12, 15) individuelle.

3. Dispositif de diagnostic radiologique suivant la revendication 1 ou 2, lequel est réalisé sous la forme d'un dispositif de diagnostic dentaire pour réaliser des tomographies panoramiques d'un maxillaire (8), selon lequel la première position de couche individuelle est définie par la face interne, et la deuxième position de couche individuelle (16) par la face externe du maxillaire (8).

4. Dispositif de diagnostic radiologique suivant la revendication 1 ou 2, lequel est réalisé sous la forme d'un dispositif de diagnostic dentaire pour réaliser des tomographies panoramiques d'un maxillaire (8), dans lequel la première position de couche (12) individuelle est définie par la face interne (13), et la deuxième position de couche (15) individuelle par la face externe (16) d'une dent (14).

5. Dispositif de diagnostic radiologique suivant l'une quelconque des revendications 1 à 4,
selon lequel l'ordinateur (6) définit la position de couche moyenne par le calcul de positions de couches individuelles dans une zone (11, 17, 18) de l'objet (1).

6. Dispositif de diagnostic radiologique suivant l'une quelconque des revendications 3 à 5,
selon lequel la position de couche moyenne (10) est adaptée à l'arcade maxillaire (9).

7. Dispositif de diagnostic radiologique suivant l'une quelconque des revendications 1 à 6,
dans lequel des signaux d'au moins une arcade maxillaire moyenne sont mémorisés dans une autre mémoire (19), l'ordinateur (6) définissant la position de couche moyenne dans au moins deux zones (11, 17, 18) différentes du maxillaire (8), et
la position de couche (10) souhaitée étant définie par une comparaison de la position de couche moyenne avec l'arcade maxillaire mémorisée.

8. Procédé pour faire fonctionner un dispositif de diagnostic radiologique suivant l'une quelconque des revendications 1 à 7, comportant les phases suivantes :
a) exploration tomographique d'un objet (1) au moyen d'un rayonnement, et génération de signaux électriques en fonction de l'ombre de rayonnement de l'objet (1),
b) mémorisation des signaux électriques pendant l'exploration tomographique,
c) exécution d'un procédé d'approximation par le calcul de positions de couches individuelles, et par leur comparaison afin de définir une position de couche (10) souhaitée, et
d) réalisation d'une tomographie de l'objet (1) dans la position de couche souhaitée.
